# EUROPÄISCHE PATENTSCHRIFT

(11) **EP 1 478 678 B1**
(45) Veröffentlichungstag und Bekanntmachung des Hinweises auf die Patenterteilung: **05.07.2006**
(21) Anmeldenummer: 03709655.9
(22) Anmeldetag: 25.02.2003
(51) Int. Cl.: C08G 65/22, C07D 301/16, C07D 301/14, C07D 303/42

(54) **PREPOLYMERE FUER NATIVE EPOXIDHARZE UND VERFAHREN ZU IHRER HERSTELLUNG**
PREPOLYMERS FOR NATIVE EPOXIDE RESINS AND METHOD FOR PRODUCING SAID PREPOLYMERS
PREPOLYMERES POUR RESINES EPOXY NATIVES ET LEUR PROCEDE DE PRODUCTION

(30) Priorität: 27.02.2002 DE 10208545
(43) Veröffentlichungstag der Anmeldung: 24.11.2004
(73) Patentinhaber: Dracowo Forschungs- und Enwicklungs Gmbh, 06766 Wolfen (DE)
(72) Erfinder: ADLER, Bernhard, 06132 Halle (DE)
(74) Vertreter: Herzog, Günter
(86) Internationale Anmeldenummer: PCT/DE2003/000595
(87) Internationale Veröffentlichungsnummer: WO 2003/072635

(56) Entgegenhaltungen:
- EP-A- 0 186 895
- WO-A-96/11919
- WO-A-96/38432
- GB-A- 789 034
- GB-A- 969 021

## Beschreibung

### Stand der Technik

Die Erfindung betrifft ein Verfahren zur Herstellung von Prepolymeren für native Epoxidharze, die industriell aber auch in den verschiedensten Bereichen des täglichen Lebens, beispielsweise als Coatingmaterialien oder Klebstoffe angewendet werden.

Epoxide werden bereits aus Industriepflanzen, insbesondere aus nativen Ölen, wegen deren biologischer Abbaubarkeit sowie ihrer nicht toxischen und nicht mutagenen Eigenschaften hergestellt. So werden beispielsweise epoxidierte Pflanzenöle zunehmend als Crosslinker für Coatingmaterialien eingesetzt (G. J. H. BUISMAAN et al.: Epoxidized Vegetable Oils and Their Use as Biodegradable Crosslinkers in Coating Formulations. 89 th AOCS Annual Meeting & Expo Abstracts Chicago 1998). Aus DE 33 20 219 ist es bekannt, ungesättigte Fettsäuren nativer Öle und Fette unter Einsatz von Persäure zu Epoxiden umzusetzen. Hierzu werden u. a. Rührkesselkaskaden im kontinuierlichen Betrieb eingesetzt. Die hierbei ablaufenden Epoxidierungsreaktionen sind durch eine Menge von Folge- und Parallelreaktionen begleitet. Eine dieser Folgereaktionen ist bei Verwendung von Peressigsäure die Bildung von Polyethern über die Zwischenstufe der Hydroxyacetate (W. SCHEIDER: Fette Seifen Anstrichmittel (1967) 573), die man aus Sicherheitsgründen, insbesondere bei der Verwendung von Rührkesseln durch Wahl der Reaktionsbedingungen, z. B. bei einer Epoxidierungstemperatur < 70° C, bemüht ist, zu unterdrücken (W. SCHNEIDER: Fette Seifen Anstrichmittel (1967) 421 und L. KOVACS: Fette Seifen Anstrichmittel (1961) 257). Denn bei der stark exotherm verlaufenden Epoxidierung ist eine Spontanzersetzung von Persäuren bei Überschreitung einer Temperaturschwelle in großvolumigen Reaktoren infolge eines eventuellen Wärmestaus nicht völlig ausschließbar (K. ECKWERT: Dissertation TU Hannover 1986). Die Absenkung der Epoxidierungstemperatur ist allerdings mit einer geringeren Raum-ZeitAusbeute verbunden.

Bekannt ist ferner, dass Produkte aus unerwünschten Folgereaktionen des obengenannten Epoxidierungsprozesses, insbesondere Polymerisate, die Abtrennung der eigentlichen Wertprodukte, nämlich der Epoxide, vom Sauerwasser erschweren, und deshalb durch den Einsatz von speziellen Inhibitoren diese Folgereaktionen technisch unterdrückt werden (DE 195 19 887 C1).

Bekannt ist auch, dass für die Startreaktion zur Bildung von Polyurethan- und Polyesterepoxiden auf Basis von epoxidiertem Leinöl Hydroxylgruppen in Form von α,β-Dihydroxy-Strukturen, die in geringen Mengen durch die Addition von Wasser an die Epoxide immer bei der Epoxidierung auftreten, notwendig sind (A. M. MOTAWIE et al.: J. Appl. Pol. Sci. (1995) 1725). Auch die Bildung älterer Epoxidharztypen aus Glycidylethern und Dicarbonsäuren als Vernetzern geht von der gleichen Initialreaktion, nämlich der Umsetzung von Hydroxylgruppen des Glycidylethers mit Dicarbonsäureanhydriden, aus (W. FISCH et al.: Über den Härtungsmechanismus der Äthoxylinharze. J. Pol. Sci. (1954) 497 - 502).

Der Erfindung liegt somit das Problem zugrunde, Prepolymere, die zur Gewinnung nativer Epoxidharze geeignet sind, ohne Sicherheitsrisiko mit einem wirtschaftlich vertretbaren Aufwand herzustellen.

### Die Erfindung und ihre Vorteile

Erfindungsgemäß wird die Aufgabe durch das im Patentanspruch 1 beschriebene Verfahren gelöst. Die folgenden Patentansprüche 2 bis 9 betreffen Variationen zu dem in Patentanspruch 1 beschriebenen grundsätzlichen Verfahren der Herstellung von Polyhydroxy-polyepoxifettsäurealkylester-ether.

Pvlyhydroxy-polyepoxi-fettsäurealkylester-ether, im Weiteren als Kurzform auch Polyhydroxyether genannt, sind eine Stoffklasse, die u. a. sowohl Oxiranringe, Etherverbindungen als auch OH-Gruppen als Vernetzungselemente besitzt, mithin ähnlich den Glycidylstrukturen aufgebaut sind, d. h. in hohem Maße den synthetischen Harzkomponenten entsprechen, aber auf völlig andere Weise synthetisiert werden. Sie zeichnen sich ferner dadurch aus, nicht toxisch oder gentoxisch zu wirken und stellen somit eine Alternative zu synthetischen Prepolymeren für Epoxidharze z. B. auf Epichlorhydrinbasis dar. Sie lassen sich gezielt herstellen, indem bei der Epoxidierung der nativen Fettsäureester die intermedier entstehenden Epoxide partiell unter katalytischer Wirkung von Wasserstoffionen oder von Lewissäuren mit OH-Gruppen haltigen Substanzen polymerisiert werden. Da bei der Epoxidierung stets ein gewisser Anteil an Hydroxystrukturen in einer Folgereaktion mit entsteht, sind einerseits Homopolymere mit sich selbst, andererseits bei Zugabe von zusätzlichen, anders strukturierten Polyolen auch gemischte copolymere Polyhydroxyether synthetisierbar.

Durch die erfindungsgemäße Verfahrensweise erhalten native (Poly)epoxide der Struktur A (siehe Formelbild 1) zur späteren Verwendung als Epoxidharzkomponenten zum besseren Reaktionsverhalten OH-Gruppen und werden dadurch also Poly(hydroxy-epoxide) gemäß Struktur A'. Sie reagieren weiter mit sich selbst unter Bildung von Oligomeren mit Ethergruppierungen, wie im Formelbild 1 der Struktur B dargestellt.

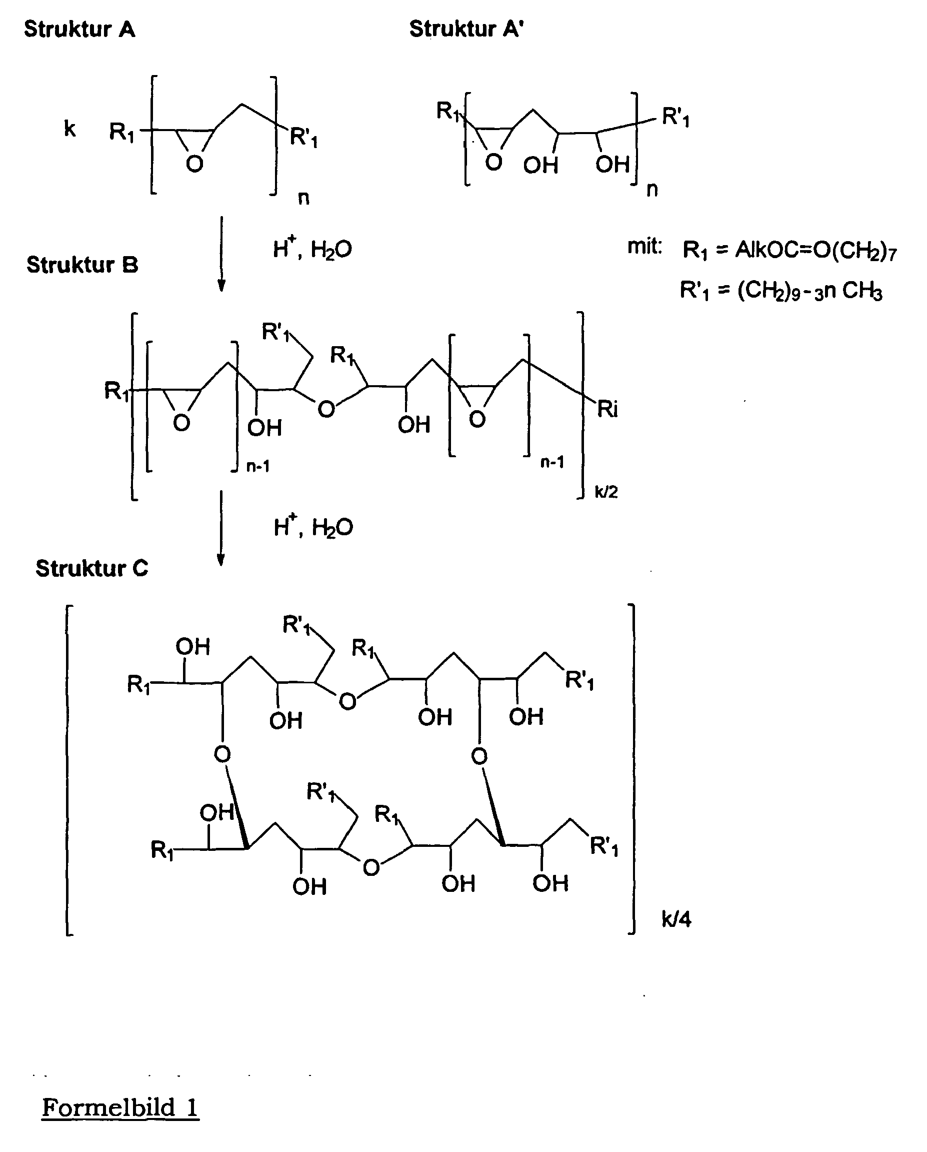

Aber genau diese Strukturforderungen sind mit den bekannten technischen Prozessen nicht realisierbar, da sich, wie oben bereits beschrieben, die eigentlichen Wertprodukte nur sehr schlecht aus der Sauerwasserphase abtrennen lassen. Aus diesem Grunde schließt sich bei dem erfindungsgemäßen Verfahren an den Prozess der Prepolymerbildung ein Abtrennprozess zu ihrer Gewinnung aus der Sauerwasserphase an. Überraschenderweise gestaltet sich eine Abtrennung der Poly(hydroxy-epoxide)- Polyhydroxyether-Gemische dann problemlos, wenn man bei ca. 65 °C unter Anwendung von sehr feinmaschigen Drahtnetzen aus Edelstahl als Koalezenzhilfen arbeitet. Der im Produkt verbleibende Sauerwasseranteil beträgt etwa 1 % und wird mittels Vakuumverdampfung restlos entfernt.

Zur technischen Herstellung von Polyhydroxyethern nativer Fettsäuregemische oder ihrer Methylesterderivate arbeitet man einmal bei Epoxidierungstemperaturen von 65 °C bis 70 ° C und mindert das Sicherheitsrisiko einer eventuellen Spontanzersetzung der in situ gebildeten Persäuren durch Einsatz einer Mikroreaktorkaskade.
Eine weitere Möglichkeit der Herstellung von Polyhydroxyethern ist dadurch gegeben, dass zunächst in an sich bekannter Weise die Polyepoxide (Struktur A in Formelbild 1) hergestellt werden und am Ende der Reaktionsphase Alkanole einmal zur Vergrößerung der Phasengrenzfläche zwischen Sauerwasser- und Produktphase zum anderen wegen ihrer katalytischen Wirkung dem Reaktionsgemisch zugegeben werden. Dies erhöht die Ausbeute sowohl an Polyhydroxyepoxiden als auch an Polyhydroxyethern.

Umgekehrt ist es aber auch möglich, durch Verringerung des Ameisensäureanteils am Ende der Reaktionsphase bei der Epoxidierung zunächst die Folgeraektionen zu blocken, so dass Polyepoxide der Struktur A entstehen. Zu beliebiger Zeit kann dann in einem zweiten Reaktionsschritt unter Einfluss von H-Ionen verdünnter Mineralsäuren bei einer Temperatur um 85 °C die Bildung von Poly(hydroxy-epoxiden) und daraus der Polyhydroxyether initiiert werden.

Eleganter als die beiden obengenannten Methoden ist ein mehrstufiger Epoxidierungs- und Polymerisationsprozess in der Mikroreaktorkaskade. Zunächst setzt man dabei nur 2,2 Doppelbindungsäquivalente pro Einsatzmolekül mit Perameisensäure um und polymerisiert bei T > 85 °C im Überschuss von Ameisensäure diese Epoxide zu partiellen Polyhydroxyethern. Anschließend epoxidiert man erneut bei Temperaturen von 60 °C < T < 65 °C die partiell gebildeten Polyhydroxyether vollständig zu den gewünschten Polyhydroxy-polyepoxifettsäurealkylester-ethern.

Eine andere Herstellungsart ist die Batch-Fahrweise in einem Graphit-Reaktor. Diesem Reaktor ist im Bypass ein Mikroreaktor mit Nachreaktor zugeordnet. Im Graphit-Reaktor herrschen die für die Epoxidierung günstigen 65 °C. Im Bypassstrom werden die für die Prepolymerenbildung erforderlichen 90 °C gehalten. Bei dieser Fahrweise entstehen Prepolymere mit ca. 2,6-Etherbrücken im statistischen Mittel. Der Vorteil dieses Verfahrens besteht in der Senkung des Gefahrenpotenzials der stark exothermen Epoxidierungsreaktion, da stets nur eine kleine Menge Reaktionsgemisch auf über 85 °C erwärmt wird.

Gemischte Polyhydroxyether lassen sich dadurch gewinnen, dass Poly(hydroxy-epoxide) in Gegenwart von Lewissäuren mit Polyolen wie z. B. Bisphenol-A copolymerisiert werden. Natürlich kann man unter katalytischer Wirkung von Lewissäuren auch die Poly(hydroxy-epoxide) mit sich selbst zu Polyhydroxyethern polymerisieren. Letztlich erfüllen Polyhydroxy-polyepoxi-fettsäurealkylester-ether mit einem bestimmten Verhältnis von OH-, Ether- und Oxirangruppen, ausgedrückt in ihren IR-spektroskopischen Extiktionen von OH-Bande bei 3425 cm⁻¹, den Etherbanden bei 1086 cm⁻¹ bzw. 1067 cm⁻¹ und Oxiranringdeformationsschwingung bei 825 - 828 cm⁻¹, die Qualitätsanforderungen als prepolymere Epoxidharzkomponenten (Tabellen 1 und 2).

Als prepolymere Polyhydroxyether reagieren die so synthetisierten Verbindungen ohne zusätzliche Wärmezuführung mit Polycarbonsäuren, wie z. B. Maleinsäure oder Phthalsäure, respektive deren Anhydriden. Mit Phosphorsäure oder ihren Pentaerythryl- bzw. Glycerylderivaten reagieren sie spontan, z. T. stark exotherm zu nativen Epoxidharzen (Formelbild 1: Strukturen, die sich aus der Vernetzung von Struktur C ergeben).

Weitere Vorteile und vorteilhafte Ausgestaltungen der Erfindung sind der nachfolgenden Beschreibung, der Zeichnung und den Ansprüchen entnehmbar.

### Beschreibung des Ausführungsbeispiels

Nachfolgend soll die Erfindung anhand mehrerer Versuchsdurchführungen näher beschrieben werden.

Alle im Weiteren abgehandelten Polyhydroxyether und Ihre Vorprodukte wurden IR- und ¹*H - NMR* spektroskopisch untersucht (Tab. 1). Ferner wurde die EO-Zahl und die Viskosität der Produkte bestimmt. Die EO-Zahl korreliert mit dem Extinktionsverhältnis der out of plan Deformationsschwingung (E1) für den Oxiranring bei 824 cm⁻¹ und der rocking-Schwingung (E2) der Methylengruppen bei 730 cm⁻¹. Es gilt: EO = 4,47 E1/E2 + 0,53. Bei Zunahme der Hydroxygruppierungen infolge Polymerisation tritt eine neue oop- Deformationsschwingung für die Hydroxystrukturen auf, die mit der Bande der Epoxidstrukturen zu einer neuen Bande bei 830 cm⁻¹ verschmilzt und die Auswertung zu erhöhten EO- Werten etwas verfälscht (Tab. 2, Spalte 5).

**Tabelle 1: Struktur- und Molekülparameter zur Charakterisierung der Produkte**

| Signal/ Molekülparameter | Signallage/Erwartungswert | Strukturparameter für |
|---|---|---|
| OH-Bande | 3475 cm⁻¹ | Poly(hydroxy-epoxide) Polyhydroxyether |
| COC-Banden | 1086 cm⁻¹ /1067 cm⁻¹)1 | Polyhydroxyether |
| oop-Deformation | 824 cm⁻¹ | Polyepoxide |
| 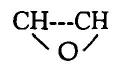 | 830 cm⁻¹ | Poly(hydroxy-epoxide) |
| CH-O-CH-Multipletts in ^{*1*}*H-NMR* | 3,4; 3,6 und 3,8 ppm | Polyhydroxyether |
| EO-Zahl | 5,1 bis 8,5 | Polyepoxide |
| | | dominant: |
| Viskosität bei 40°C | > 80 cSt | Polyepoxide |
| | >160 cSt | Poly(hydroxy-epoxide) |
| | >650 cSt | Polyhydroxyether |

| | | |
|---|---|---|
| 1 zur Routineauswertung kommt die mit der Glycidrestbande bei 1103 cm⁻¹ sich vereinigenden 3 Banden | | |

**Tabelle 2: IR-Extinktionsverhältnisse für Poly(hydroxyepoxide) sowie daraus hergestelltem prepolymeren Polyhydroxyether**

| Chargen / Vorschrift | *E*_{*v(OH)*} / *E*_{*v(COC)*} | *E*_{*v(COC)*} / *E*_{*v(EO)*} | *E*_{*v(COC)*} / *E*_{*v(EO)*} | *E*_{*v(EO)*} / *E*_{*p(CH*_{*2*}*)*} |
|---|---|---|---|---|
| 85/**A**)1 | 0,38 | 0,71 | 2,6 | 1,42 |
| V54/**A'** | 0,2 | 0,4 | 2,4 | 1,36 |
| 62/**B** | 0,5 | 0,3 | 3,3 | 1,2 |
| 91/**C** | 0,34 | 0,7 | 2,5 | 1,44)2 |
| 74/**D** | 0,34 | 0,63 | 2,0 | 1,4)2 |
| 85/10 **E** | 0,83 | 1,7 | 1,34 | 1,56)2 |
| 85/9 **F** | 2,2 | 0.88 | 2,5 | 1,21 |

| | | | | |
|---|---|---|---|---|
| )1 Mittelwerte, )2 oop Deformationsschwingung bei 824 cm⁻¹ von Polyhydroxystrukturen von oop Deformationsschwingung bei 830 cm⁻¹ überlagert | | | | |

### A Herstellung von Poly(hydroxy-epoxiden)

1 Mol Leinöl werden mit 5 Mol Wasserstoffperoxid in Gegenwart von 3,25 Mol Ameisensäure bei konstanter Temperatur von 65 °C umgesetzt. Nach beginnendem Abfall der zunächst spontanen Wärmeentwicklung wird das Reaktionsgemisch noch 30 min bei 65 °C gehalten und danach über ein feinmaschiges Drahtnetz aus Edelstahl bei der gleichen Temperatur vom Sauerwasser getrennt. Die verbleibenden ca.1 % Sauerwasser werden im Vakuumverdampfer abgezogen bis das Syntheseprodukt transparent ist. Die gebildeten Polyhydroxypolyepoxi-fettsäureglycidester besitzen folgende Parameter: Die EO-Zahl beträgt 6,2, die Viskosität 170 cST bei 40 °C.

### A' Herstellung von Poly(hydroxy-epoxiden) mittels Mikroreaktorkaskade

6 Mol Leinöl werden mit 35 Mol Wasserstoffperoxid und 18 Mol Ameisensäure in einer Mikroreaktokaskade mit einer Fließgeschwindigkeit von 11/min bei 70 °C zur Reaktion gebracht. Nach Durchlaufen der Mischstrecke des Mikroreaktors wird das Produkt unter langsamem Abdampfen der Ameisensäure bei 65 °C 2 h nachgerührt. Die Abtrennung des Sauerwassers erfolgt wie unter A beschrieben. Die EO-Zahl beträgt 6,2; die Viskosität des Produktes bei 40 °C 87 cST.

### B Polymerisation unter Einsatz von Methanol als Phasenvermittler

1 Mol Leinöl wird mit 6 Mol Wasserstoffperoxid und 6 Mol Ameisensäure bei 65° C umgesetzt. Bei Abfall der Temperatur wird 1 h bei 85 °C nachgeheizt. Vor dem Nachheizen gibt man zum Reaktionsgemisch ca. 4 ml Methanol zu und hält durch Nachheizen die Temperatur 20 min bei 65 °C. Die Aufarbeitung erfolgt wie unter **A** beschrieben. Die EO-Zahl beträgt 6,0; die Viskosität des Produktes 448 cST.

### C Polymerisation unter Zugabe von Phosphorsäure

1 Mol Drachenkopföl wird mit 6 Mol Wasserstoffperoxid und 3 Mol Ameisensäure und 10 ml 85% Phosphorsäure bei 65 °C umgesetzt. Die Aufarbeitung erfolgt wie unter **A** beschrieben. Die Viskosität bei 40 °C beträgt 780 cST; die EO-Zahl 8,5.

### D Vorpolymerisation mit anschließender Epoxidierung

1 Mol Leinöl werden mit 2,5 Mol Wasserstoffperoxid und 3 Mol Ameisensäure bei 65 °C zur Reaktion gebracht. Bei Abfall der Temperatur in einem Nachrührbehälter wird 5 min bei 85 °C nachgeheizt. Danach werden zur vollständigen Epoxidierung weitere 3 Mol Wasserstoffperoxid und 3 Mol an Ameisensäure der Reaktionslösung zugesetzt und bei Temperaturen < 65 °C zur Reaktion gebracht. Die Aufarbeitung erfolgt wie unter A beschrieben. Die Viskosität bei 40 °C beträgt 281 cST, die EO-Zahl 6.9.

### E Umsetzung von Poly(hydroxyepoxiden) mit Polyolen zu gemischten Polyethern

98 g Polyhydroxyepoxid nach Vorschrift **A** werden mit 2 g Bisphenol-A unter katalytischer Wirkung von Zinntetrachlorid in getrocknetem Aceton gelöst und 30 min bei 85 °C umgesetzt. Das Aceton wird dabei ausgetrieben. Das Epoximultiplett bei 3,1 ppm im ¹*H - NMR* -Spektrum nimmt ca. um 1/3 ab. ; bei 3,4 und 3,74 ppm erscheinen bandenförmige Multipletts der Etherstrukturen.

### F Umsetzung von Polyhydroxypolyepoxiden mit sich selbst in Gegenwart von Lewissäuren

1 Mol Leinöl werden mit 5 Mol Wasserstoffperoxid in Gegenwart von 3,25 Mol Ameisensäure bei 65 °C umgesetzt; das Sauerwasser ebenfalls bei 65 °C abgetrennt. Die Viskosität beträgt 176 cST. Fügt man diesem Gemisch aus Polyepoxiden und Poly(hydroxyepoxiden) bei 85 °C 1/1000 Mol Zinntetrachlorid in 5 ml i-Propanol gelöst hinzu, steigt die Viskosität nach 15 min auf 1155 cSt an.

### G Umsetzung der Polyhydroxyether mit Vernetzern

3 g Prepolymeres nach **A** bis **F** werden mit 0,2 g Glycerophosphat (Pentaerythritphosphat), was aus 1 Mol Glycerin (Pentaerythrit) und 1,5 Mol 85% Phosphorsäure unter Vakuum bei 15 - 20 mbar und Temperaturen von 75 - 95 °C hergestellt wird, zur Reaktion gebracht. Die Topfzeit beträgt ca. 15'. Sie kann bei Zugabe von Essigsäureanhydrid oder Zugabe von 0,1 - 20 % Dicarbonsäureanhydriden je nach gewünschtem Verwendungszweck des Polymerisates verkürzt werden. Die Aushärtung ist variabel einstellbar und liegt bei 2 - 6 h

Alle in der Beschreibung, den nachfolgenden Ansprüchen und der Zeichnung dargestellten Merkmale können sowohl einzeln als auch in beliebiger Kombination miteinander erfindungswesentlich sein.

## Patentansprüche

1. Verfahren zur Herstellung von Prepolymeren für native Epoxidharze unter Verwendung nativer Fettsäurealkylester, beispielsweise Polyhydroxy-polyepoxi-fettsäurealkylester-ether, im Weiteren als Kurzform auch Polyhydroxyether genannt
**dadurch gekennzeichnet,**
**dass** die Fettsäurealkylester unter Einfluss von Perameisensäure bei einer Temperatur von 70 °C bis 90 °C mit Wasserstoffperoxid epoxidiert werden, die entstandenen Produktgemische bei einer Temperatur von ca. 65 °C unter Anwendung feinmaschiger Drahtnetze vom Sauerwasser getrennt werden und der verbleibende Rest von Sauerwasser mittels Vakuumverdampfung entfernt wird.

2. Verfahren nach Anspruch 1,
**dadurch gekennzeichnet,**
**dass** bei der Epoxidierung zunächst primäre Polyepoxide der Struktur A entstehen, die in einer Folgereaktion bei den o. g. Temperaturen über eine Zwischenstufe der Polyhydroxyepoxide der Struktur A' in Polyhydroxyether der Struktur B übergehen (siehe Formelbild 1).

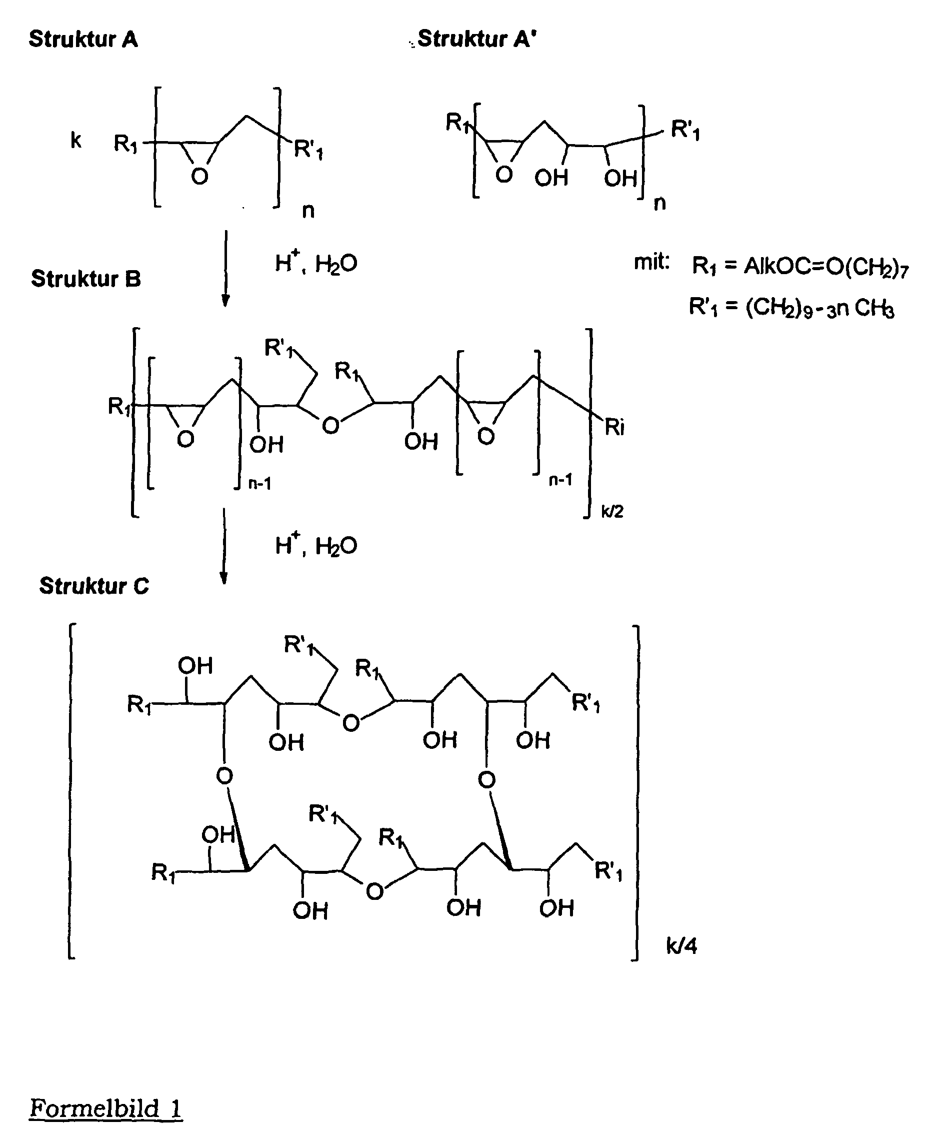

3. Verfahren nach Anspruch 1 und 2,
**dadurch gekennzeichnet,**
**dass** gegen Reaktionsende Alkanole als Phasenvermittler zwischen Sauerwasserphase und Epoxidphase zugegeben werden.

4. Verfahren nach Anspruch 1 und 2,
**dadurch gekennzeichnet,**
**dass** gegen Reaktionsende der Ameisensäureanteil verringert wird, wobei **dadurch** zunächst die Folgereaktionen zur Zwischenstufe A' geblockt bleiben, und in einem zweiten Reaktionsschritt zu beliebiger Zeit unter Zugabe einer verdünnten Schwefel- oder Phosphorsäure die Bildung der Polyhydroxyepoxide der Struktur A' und daraus weiter die Bildung der Polyhydroxyether initiiert werden.

5. Verfahren nach Anspruch 1,
**dadurch gekennzeichnet,**
**dass** bei den Fettsäurealkylestern zunächst partiell nur 2, 2 Doppelbindungsäquivalente pro Molekül epoxidiert und sofort bei Temperaturen > 85 °C unter Einfluss des Sauerwassers vollständig verethert werden und anschließend die partiell gebildeten Polyhydroxyether bei Temperaturen < 65 °C vollständig durchepoxidiert werden.

6. Verfahren nach Anspruch 1,
**dadurch gekennzeichnet,**
**dass** die Epoxidierung in einem Graphit-Reaktor bei ca. 65 °C erfolgt und die Prepolymere zeitgleich in einem dem Graphit-Reaktor im Bypass angeschlossenen Mikroreaktor bei ca. 90 °C entstehen.

7. Verfahren nach Anspruch 1,
**dadurch gekennzeichnet,**
**dass** primär hergestellte Polyhydroxyepoxide durch Zugabe von Lewissäure mit sich selbst zu Polyhydroxyethern poiymerisieren.

8. Verfahren nach Anspruch 2,
**dadurch gekennzeichnet,**
**dass** zur Herstellung copolymerer Polyhydroxyether die Poly(hydroxy-epoxide) mit anders strukturierten Polyhydroxyden und Lewissäure versetzt werden.

9. Verfahren nach Anspruch 1,
**dadurch gekennzeichnet,**
**dass** Polyhydroxyether aus freien Fettsäuren oder ihren Methylestern hergestellt werden.

## Claims

1. Process for producing prepolymers for native epoxy resins using native fatty acid alkyl esters, such as e.g. polyhydroxy-polyepoxyfatty acid alkyl ester ethers, hereinafter also referred to in the abbreviated form as polyhydroxyethers,
**characterised in that** the fatty acid alkyl esters are epoxidised under the influence of performic acid at a temperature of 70°C to 90°C with hydrogen peroxide, the product mixtures formed are separated from the acidic water at a temperature of approx 65°C using fine mesh wire netting and the remainder of the acidic water is removed by vacuum evaporation.

2. Process according to claim 1
**characterised in that**
during the epoxidation, primary polyepoxides with structure A are initially formed which, in a subsequent reaction at the above-mentioned temperatures, change, via an intermediate stage of the polyhydroepoxides with structure A, into the polyhydroxyether with structure B (compare graphic formula 1).

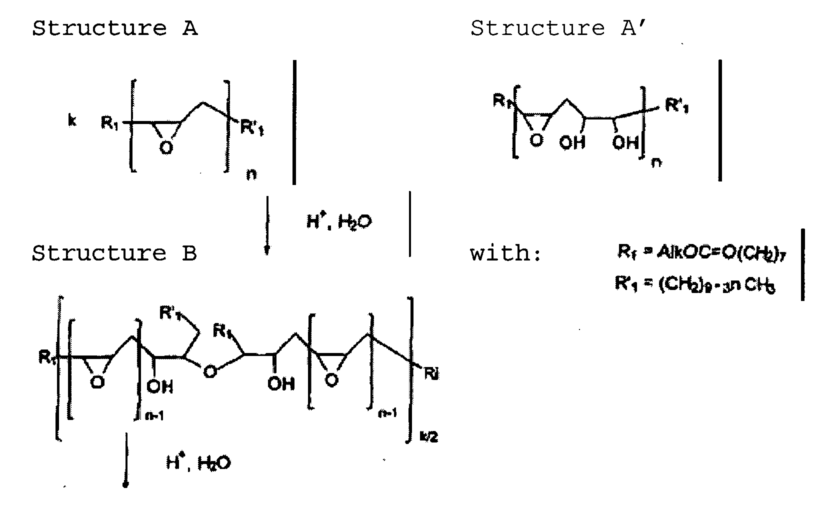

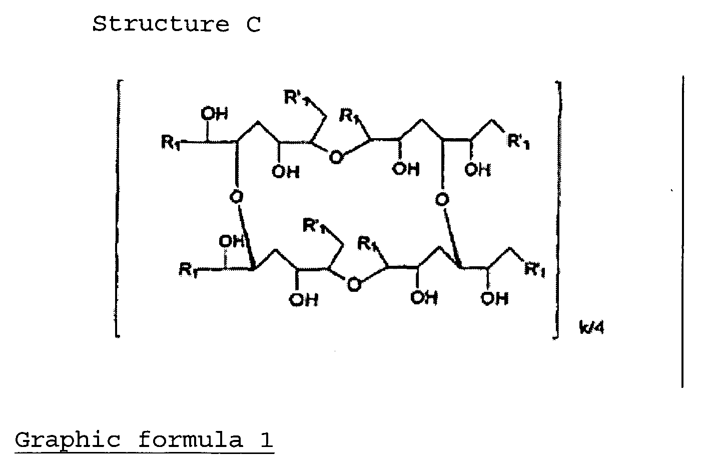

3. Process according to claim 1 and 2
**characterised in that**
towards the end of the reaction, alkanols are added as phase mediators between the acidic water phase and the epoxide phase.

4. Process according to claim 1 and 2
**characterised in that**,
towards the end of the reaction, the formic acid portion is reduced as a result of which the subsequent reaction to the intermediate stage A' remain initially blocked and, in a second reaction step, the formation of the polyhydroxyepoxides with structure A' takes place at any desired time with an addition of a dilute sulphuric or phosphoric acid, and the formation of the polyhydroxyethers is then initiated therefrom.

5. Process according to claim 1
**characterised in that**
in the fatty acid alkyl esters, initially only 2,2 double bond equivalents per molecule are partially oxidised and fully etherified immediately at temperature of > 85°C under the influence of the acidic water and subsequently the partially formed polyhydroxyethers are completely epoxidised through at temperatures of < 65°C.

6. Process according to claim 1,
**characterised in that**
the epoxidation takes place in a graphite reactor at approx. 65°C and the prepolymers are formed simultaneously in a microreactor connected to the graphite reactor in the bypass at approx. 90°C.

7. Process according to claim 1
**characterised in that**
polyhydroxyepoxides formed primarily are polymerised with themselves by the addition of Lewis acid to form polyhydoxyethers.

8. Process according to claim 2
**characterised in that**
for the production of copolymeric polyhydroxyethers, the poly[hydroxyepoxides] are mixed with differently structured polyhydroxides and Lewis acids.

9. Process according to claim 1
**characterised in that**
polyhydroxyethers are produced from free fatty acids or their methyl esters.

## Revendications

1. Procédé de fabrication de prépolymères pour résines époxy naturelles en utilisant des alkylesters naturels d'acide gras, par exemple des alkylesters-éthers polyhydroxy-polyépoxy d'acides gras, appelés ci-après de manière abrégée aussi polyhydroxyéthers,
**caractérisé en ce que**
les alkylesters d'acide gras sont époxydés sous l'influence d'acide performique à une température de 70° C à 90° C avec du peroxyde d'hydrogène, les mélanges de produits obtenus sont séparés de l'eau acide à une température d'environ 65° C en utilisant des filets de fil de fer à mailles fines et le résidu d'oxygène est enlevé de l'eau acide par vaporisation sous vide.

2. Procédé selon la revendication 1,
**caractérisé en ce que**,
lors de l'époxydation, on obtient d'abord des polyépoxys primaires de structure A qui se transforment en une réaction successive aux températures précitées en passant par un stade intermédiaire de polyhydroxyépoxys de structure A' en polyhydroxyéthers de structure B (voir formule 1).

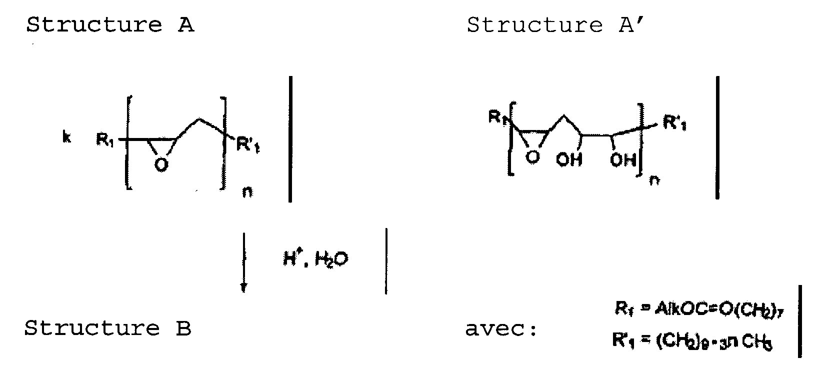

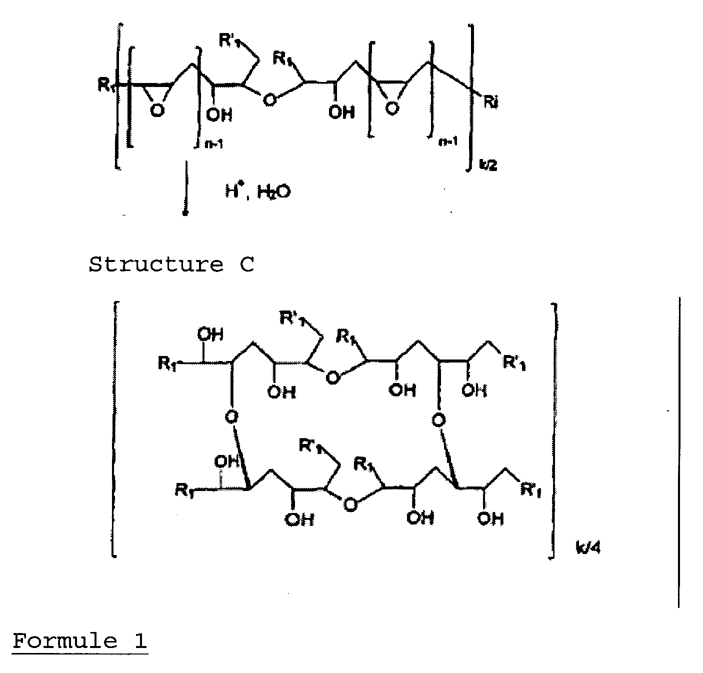

3. Procédé selon les revendications 1 et 2,
**caractérisé en ce que**,
vers la fin de la réaction, on ajoute des alcanols servant d'agents de phase entre la phase oxygène et la phase époxy.

4. Procédé selon les revendications 1 et 2,
**caractérisé en ce que**,
vers la fin de la réaction, on réduit la proportion d'acide performique, ce qui a d'abord pour effet que les réactions successives vers le stade intermédiaire A' restent bloquées et que, pendant une deuxième étape réactionnelle, à tout moment, en ajoutant un acide sulfurique ou phosphorique dilué, la formation des polyhydroxyépoxys de structure A' et donc la formation des polyhydroxyéthers sont initiées.

5. Procédé selon la revendication 1,
**caractérisé en ce que**,
pour les alkylesters d'acides gras, on n'époxyde d'abord partiellement que des 2, 2 équivalents de double liaison par molécule et on éthérifie immédiatement complètement à des températures > 85° C sous l'influence de l'eau acide, puis on époxyde complètement les polyhydroxyéthers partiellement formés à des températures < 65° C.

6. Procédé selon la revendication 1,
**caractérisé en ce que**
l'époxydation a lieu dans un réacteur à graphite à environ 65° C et les prépolymères apparaissent en même temps à environ 90° C dans un microréacteur raccordé en dérivation au réacteur à graphite.

7. Procédé selon la revendication 1,
**caractérisé en ce que**
les polyhydroxyépoxys préparés au niveau primaire polymérisent par eux-mêmes en polyhydroxyéthers par ajout d'acide Lewis.

8. Procédé selon la revendication 2,
**caractérisé en ce que**,
pour préparer des polyhydroxyéthers copolymères, on transforme les poly(hydroxy-époxys) avec d'autres polyhydroxys autrement structurés et de l'acide Lewis.

9. Procédé selon la revendication 1,
**caractérisé en ce que**
les polyhydroxyéthers sont préparés à partir d'acides gras libres ou leurs méthylesters.
